# EUROPEAN PATENT APPLICATION

(11) **EP 4 786 580 A1**
(43) Date of publication of application: **05.08.2026**
(21) Application number: 24881647.2
(22) Date of filing: 23.10.2024
(51) Int. Cl.: C12N 5/071, C12N 5/09, C12N 5/077, C12N 5/02, C12Q 1/02

(54) **METHOD FOR CONSTRUCTING VASCULARIZED ORGANOID SPHEROID MODEL, VASCULARIZED ORGANOID SPHEROID MODEL AND USE THEREOF**

(30) Priority: 23.10.2023 CN 202311376915; 23.10.2023 CN 202311377344
(71) Applicant: Jiangsu Avatarget Biotechnology Co., Ltd., Nanjing, Jiangsu 211800 (CN)
(72) Inventor: ZHANG, Jing, Nanjing, Jiangsu 211800 (CN); YANG, Feili, Nanjing, Jiangsu 211800 (CN); ZHUANSUN, Xuemei, Nanjing, Jiangsu 211800 (CN); WEI, Yuan, Nanjing, Jiangsu 211800 (CN); LIU, Xueqiang, Nanjing, Jiangsu 211800 (CN)
(74) Representative: Winter, Brandl - Partnerschaft mbB
(86) International application number: PCT/CN2024/126737
(87) International publication number: WO 2025/087283

(57) **Abstract**

The present invention provides a method for constructing a vascularized organoid spheroid model, a vascularized organoid spheroid model, and use thereof. The method includes: S100: preparing or obtaining an organoid, digesting the organoid to obtain organoid cells, and obtaining fibroblasts and vascular endothelial cells; S200: mixing the organoid cells, the fibroblasts and the vascular endothelial cells, and resuspending them with a culture medium to obtain an organoid multicellular suspension; and S300: inoculating the organoid multicellular suspension into a culture chamber having a U-shaped bottom, or a culture well having a U-shaped bottom, and performing spheroid culture, allowing self-assembling to form the vascularized organoid spheroid model. The present disclosure uses co-culture of organoid cells, fibroblasts and vascular endothelial cells, and also uses a low-adhesion U-shaped well plate to form microspheres, so as to prepare the vascularized organoid spheroid model.

## Description

### TECHNICAL FIELD

The present disclosure relates to the field of bioengineering technology, and in particular to a method for constructing a vascularized organoid spheroid model, a vascularized organoid spheroid model, and use thereof.

### BACKGROUND

Cancer is a highly structured and complex ecosystem, including tumor cells and a large number of non-tumor cells, all embedded in an extracellular matrix. The tumor microenvironment (TME) includes various immune cells, cancer-associated fibroblasts, endothelial cells, pericytes, and various other tissue-resident cells. This complex environment directly or indirectly affects tumor cell phenotypes through complex mutual signal transduction mechanisms mediated by soluble factors, cell-cell contacts, and extracellular matrix (ECM) remodeling, leading to high heterogeneity of tumors. Cells in the tumor microenvironment are involved in every stage of cancer progression, affecting tumor initiation, progression, invasion, intravasation, and metastatic dissemination and growth. Cells in the tumor microenvironment and the molecules they secrete are considered to play a key role in the pathogenesis of cancer, representing attractive therapeutic targets. The complex interaction between tumor cells and mediators is crucial for the rational development of effective anti-cancer drugs.

The heterogeneity of cancer is a major contributor to variations in patient response to treatment, including treatment resistance. This complexity has become a considerable obstacle to discovering specific mechanisms of treatment failure. Patient-specific tumor organotypic models can change the understanding of cancer heterogeneity and its impact on personalized medicine. These advances are partly attributed to the ability of organoid models to stably preserve the genetic, proteomic, morphological and drug characteristics of the parental tumor in vitro, while also providing unprecedented genomic and environmental manipulation. However, tumor organoid models are difficult to fully characterize the original tissue because they often lack a complete microenvironment. Tumor organoids and the tumor microenvironment can be likened to "seed and soil". Constructing a tumor organoid model with a microenvironment for drug testing provides test results that can more closely mimic the real response of the human body to drug action.

Angiogenesis inhibitors are an important class of anti-tumor drugs. In 1971, Professor Folkman proposed the theory of tumor angiogenesis, pointing out that both local tumor growth and distant metastasis are inseparable from the generation of tumor neovasculature. The growth and metastasis of solid tumors involve a transition from the prevascular phase to the vascular phase. Through tumor angiogenesis inhibitors, the formation of the tumor neovascularization factor VEGF can be inhibited, thereby inducing natural apoptosis of vascular endothelial cells and destroying the tumor neovascular network. However, traditional in vitro models are difficult to detect the anti-tumor effect of such drugs. Tumor models such as tumor cell models, tumor organoid models, and tumor aggregate models only contain tumor cells and do not respond to angiogenesis inhibitor drugs, while vascular models can only reflect the effect on angiogenesis but cannot further reflect the effect on tumors.

### SUMMARY

In view of this, the present disclosure provides a method for constructing a vascularized organoid spheroid model, a vascularized organoid spheroid model, and use thereof. The vascularized organoid spheroid model constructed by the method described in the present disclosure has a dense structure and high biomimetic fidelity, more closely mimicking the real response of the human body to drug action. It can be applied to the screening of anti-tumor drugs and the sensitivity test of anti-tumor drugs, as well as the toxicity test of non-tumor drugs.

In a first aspect, the present disclosure provides a method for constructing a vascularized organoid spheroid model, wherein the method includes: S100: preparing or obtaining an organoid, wherein the organoid is a primary organoid or a passaged organoid; digesting the primary organoid or the passaged organoid to obtain organoid cells; and obtaining fibroblasts and vascular endothelial cells; S200: mixing the organoid cells, the fibroblasts and the vascular endothelial cells, and resuspending them with a culture medium to obtain an organoid multicellular suspension; and S300: inoculating the organoid multicellular suspension into a culture chamber or a culture well, and performing spheroid culture, allowing self-assembly to form the vascularized organoid spheroid model.

The organoid is an organoid constructed from tissue extraction or an organoid differentiated from iPS stem cells. The organoid may be a primary organoid or an organoid to be passaged.

Exemplarily, in some embodiments, the organoid multicellular suspension is inoculated into a culture chamber for spheroid culture, wherein the culture chamber has a U-shaped bottom. In other embodiments, the organoid multicellular suspension is inoculated into a culture well for spheroid culture, wherein the culture well has a U-shaped bottom. Preferably, the culture chamber and/or the culture well is a low-adhesion U-shaped culture chamber and/or a low-adhesion U-shaped culture well, to facilitate subsequent self-assembly spheroid culture.

In the method for constructing a vascularized organoid spheroid model described in the present disclosure, firstly, organoid cells obtained by digesting a primary organoid or a passaged organoid are mixed with fibroblasts and vascular endothelial cells in appropriate proportions, and then multi-stage spheroid culture is performed sequentially. During the culture process, a culture vessel with a U-shaped bottom is used to facilitate the formation of a tight spheroid structure. On one hand, the organoid cells used in the present disclosure can truly reflect the physiological characteristics of tumor tissue and non-tumor tissue; on the other hand, the organoid cells can proliferate and be passaged, so that the prepared vascularized organoid spheroid model has superior biomimetic fidelity. Moreover, in the present disclosure, by co-culturing an appropriate number of fibroblasts and an appropriate number of vascular endothelial cells with organoid cells, an organoid microenvironment composed of fibroblast structures and microvessels can be obtained, which further improves the biomimetic fidelity of the prepared vascularized organoid spheroid model, so that the prepared vascularized organoid spheroid model can better represent the original tissue. In the construction method of the present disclosure, spheroid culture in combination with cell culture scaffold materials (such as hydrogel, matrigel, and other cell culture gels) are used to facilitate the observation of cell migration during subsequent drug sensitivity testing and to study tumor invasion.

In some embodiments, a total cell count of organoid cells, fibroblasts and vascular endothelial cells ranges from 5,000 to 2×10⁴, and the diameter of the constructed vascularized organoid spheroid model ranges from 250 µm to 800 µm. A total cell count within the aforementioned range, combined with a suitable model diameter, facilitates the formation of a necrotic core inside the model. This is beneficial for the gradient increase of the extent of cell proliferation from the inside to the outside of the model and the gradual increase of oxygen content from the inside to the outside, resulting in superior biomimetic fidelity. Preferably, the diameter of the constructed vascularized organoid spheroid model ranges from 300 µm to 600 µm. More preferably, the diameter of the constructed vascularized organoid spheroid model ranges from 350 µm to 450 µm.

In some embodiments, the organoid is a primary tumor organoid or a passaged tumor organoid. The primary tumor organoid or the passaged tumor organoid is digested into single cells to obtain tumor organoid cells. The fibroblasts include tumor-associated fibroblasts. The cell number ratio of the tumor organoid cells, the tumor-associated fibroblasts and the vascular endothelial cells is 1: (0.1-1): (0.05-1). When the cell number ratio of the three types of cells is within the above range, the constructed vascularized organoid spheroid model has a denser structure and tighter intercellular connections, which is beneficial for improving biomimetic fidelity.

In the present disclosure, unless otherwise specified, the culture medium includes at least components for culturing organoid cells and components for culturing vascular endothelial cells.

In some embodiments, the culture medium is a mixed culture medium, and the mixed culture medium includes at least a first culture medium for culturing the organoid cells and a second culture medium for culturing the vascular endothelial cells. In the mixed culture medium, the mass ratio of the first culture medium to the second culture medium is 1: (0.5-2). In this case, it is more conducive to the co-culture of organoid cells, fibroblasts and vascular endothelial cells, and is beneficial to the growth and reproduction of the vascularized organoid spheroid. Preferably, the mass ratio of the first culture medium to the second culture medium is 1: (0.8-1.2).

In some embodiments, the spheroid culture includes a first-stage spheroid culture and a second-stage spheroid culture performed sequentially. The first-stage spheroid culture includes: inoculating the organoid multicellular suspension into the culture chamber with a U-shaped bottom or the culture well with a U-shaped bottom for aggregation treatment to aggregate the cells in the organoid multicellular suspension, and then performing culture for 1 to 5 days after placement in a culture environment, to obtain an organoid spheroid model intermediate with clear boundaries and microvessels, enabling the second-stage spheroid culture to be performed subsequently. Wherein, the culture time of the first-stage spheroid culture is preferably 2 to 3 days. It should be noted that in the present disclosure, unless otherwise specified, the culture environment refers to the standard culture environment of 37°C and 5% CO₂. Preferably, the aggregation treatment includes centrifugation at 950 rpm to 1,050 rpm for 5 min to 6 min.

In some embodiments, the second-stage spheroid culture includes: discarding the original culture medium in the culture chamber or the culture well containing the organoid spheroid model intermediate, adding a cell culture scaffold material, adding the mixed culture medium after gelation, and continuing to perform culture for 1 to 2 days after placement in a culture environment. Preferably, the culture time of the second-stage spheroid culture is 1 day.

In other embodiments, the second-stage spheroid culture includes: discarding the original culture medium in the culture chamber or the culture well; adding a cell culture scaffold material; transferring a resulting mixture to a culture chamber of a drug-sensitive chip or a culture well of a drug-sensitive chip, wherein the culture chambers of the drug-sensitive chip and the culture wells of the drug-sensitive chip have U-shaped bottoms and are interconnected; adding the mixed culture medium after gelation; and performing dynamic culture after placement in a culture environment, wherein the culture time of the second-stage spheroid culture is 1 to 2 days. Preferably, the culture time of the second-stage spheroid culture is 1 day.

In some embodiments, the dynamic culture includes: performing flow perfusion culture at a speed of rocking 3° to 10° every 15 min to 120 min in a culture environment. Exemplarily, in some embodiments, the perfusion culture is performed at a rocking angle of 3°/120 min in the culture environment. In other embodiments, the perfusion culture is performed at a rocking angle of 7°/30 min in the culture environment. In yet other embodiments, the perfusion culture is performed at a rocking angle of 7°/15 min in the culture environment.

In some embodiments, the organoid is selected from any one of a primary lung cancer organoid, a lung cancer organoid differentiated from iPS stem cells, or a passaged lung cancer organoid. The primary lung cancer organoid, the lung cancer organoid differentiated from iPS stem cells or the passaged lung cancer organoid is digested into single cells to obtain lung cancer organoid cells. The fibroblasts include lung cancer-associated fibroblasts. The cell number ratio of the lung cancer organoid cells, the lung cancer-associated fibroblasts and the vascular endothelial cells is 1: (0.4-0.8): (0.1-0.5). In this case, the mixed culture medium includes at least a first culture medium for culturing the lung cancer organoid cells and a second culture medium for culturing the vascular endothelial cells, and the mass ratio of the first culture medium to the second culture medium is 1: (0.5-2).

Exemplarily, the lung cancer organoid cells, the lung cancer-associated fibroblasts and the vascular endothelial cells are mixed according to a cell number ratio of 1: (0.4-0.8): (0.1-0.5) and resuspended with the above mixed culture medium to obtain a lung cancer organoid multicellular suspension. The lung cancer organoid multicellular suspension is inoculated into a culture chamber or a culture well with a U-shaped bottom to perform the two-stage spheroid culture. Alternatively, after the first-stage spheroid culture is completed, the lung cancer organoid multicellular suspension is transferred to a culture chamber of a drug-sensitive chip or a culture well of a drug-sensitive chip. After gelation, the above mixed culture medium is added, and the drug-sensitive chip is placed into a culture environment for the second-stage spheroid culture.

In some embodiments, the organoid is a primary colorectal cancer organoid or a passaged colorectal cancer organoid. The primary colorectal cancer organoid or the passaged colorectal cancer organoid is digested into single cells to obtain colorectal cancer organoid cells. The fibroblasts include colorectal cancer-associated fibroblasts. The cell number ratio of the colorectal cancer organoid cells, the colorectal cancer-associated fibroblasts and the vascular endothelial cells is 1: (0.1-0.8): (0.2-0.6). In this case, the mixed culture medium includes at least a first culture medium for culturing the colorectal cancer organoid cells and a second culture medium for culturing the vascular endothelial cells, and the mass ratio of the first culture medium to the second culture medium is 1: (0.5-2).

Exemplarily, the colorectal cancer organoid cells, the colorectal cancer-associated fibroblasts and the vascular endothelial cells are mixed and resuspended with the above mixed culture medium to obtain a colorectal cancer organoid multicellular suspension. The colorectal cancer organoid multicellular suspension is inoculated into a culture chamber or a culture well with a U-shaped bottom to perform the two-stage spheroid culture. Alternatively, after the first-stage spheroid culture is completed, the colorectal cancer organoid multicellular suspension is transferred to a culture chamber of a drug-sensitive chip or a culture well of a drug-sensitive chip. After gelation, the above mixed culture medium is added, and the drug-sensitive chip is placed into a culture environment for the second-stage spheroid culture.

In some embodiments, the organoid is a primary pancreatic cancer organoid or a passaged pancreatic cancer organoid. The primary pancreatic cancer organoid or the passaged pancreatic cancer organoid is digested into single cells to obtain pancreatic cancer organoid cells. The fibroblasts include pancreatic cancer-associated fibroblasts. The cell number ratio of the pancreatic cancer organoid cells, the pancreatic cancer-associated fibroblasts and the vascular endothelial cells is 1: (0.2-0.7): (0.2-0.7). In this case, the mixed culture medium includes at least a first culture medium for culturing the pancreatic cancer organoid cells and a second culture medium for culturing the vascular endothelial cells, and the mass ratio of the first culture medium to the second culture medium is 1: (0.5-2).

Exemplarily, the pancreatic cancer organoid cells, the pancreatic cancer-associated fibroblasts and the vascular endothelial cells are mixed and resuspended with the above mixed culture medium to obtain a pancreatic cancer organoid multicellular suspension. The pancreatic cancer organoid multicellular suspension is inoculated into a culture chamber or a culture well with a U-shaped bottom to perform the two-stage spheroid culture. Alternatively, after the first-stage spheroid culture is completed, the pancreatic cancer organoid multicellular suspension is transferred to a culture chamber of a drug-sensitive chip or a culture well of a drug-sensitive chip. After gelation, the above mixed culture medium is added, and the drug-sensitive chip is placed into a culture environment for the second-stage spheroid culture.

In some embodiments, the organoid is a primary non-tumor organoid or a passaged non-tumor organoid. The primary non-tumor organoid or the passaged non-tumor organoid is digested into single cells to obtain non-tumor organoid cells. The non-tumor organoid is selected from any one of a liver organoid, a lung organoid, a colorectal organoid or a pancreatic organoid. The fibroblasts include associated fibroblasts, for example, liver-associated fibroblasts, lung-associated fibroblasts, colorectal-associated fibroblasts or pancreatic-associated fibroblasts. The non-tumor organoid cells, the associated fibroblasts and the vascular endothelial cells are mixed according to a cell number ratio of 1: (1-2): (1-2) and resuspended with a mixed culture medium to obtain a non-tumor organoid multicellular suspension. In this case, the mixed culture medium includes at least a first culture medium for culturing the non-tumor organoid cells and a second culture medium for culturing the vascular endothelial cells, and the mass ratio of the first culture medium to the second culture medium is 1: (0.5-2).

In some embodiments, the non-tumor organoid multicellular suspension is inoculated into a culture chamber or a culture well with a U-shaped bottom to perform the two-stage spheroid culture. In other embodiments, after the first-stage spheroid culture is completed, the non-tumor organoid multicellular suspension is transferred to a culture chamber of a drug-sensitive chip or a culture well of a drug-sensitive chip. After gelation, a mixed culture medium is added, and the drug-sensitive chip is placed into a culture environment for the second-stage spheroid culture.

In the construction method of the present disclosure, firstly, the organoid multicellular suspension is inoculated into a U-shaped culture chamber or a U-shaped culture well, and then the first-stage spheroid culture and the second-stage spheroid culture are performed sequentially. Through the first-stage spheroid culture, an organoid spheroid model intermediate with clear boundaries and microvessels can be obtained. Subsequently, a cell culture scaffold material is further added to the U-shaped culture chamber or U-shaped culture well containing the organoid spheroid model intermediate. After gelation, the second-stage spheroid culture is performed. The second-stage spheroid culture may be static culture directly placed in a culture environment or dynamic culture placed in a culture environment, which can be selected according to the actual situation and is not limited by the present disclosure. Through the second-stage spheroid culture with matrix addition in the present disclosure, the microvessels continue to grow to form a large-area complex network, which is beneficial for observing cell migration during subsequent drug testing and provides better biomimetic fidelity. In some embodiments, the U-shaped culture chamber is a low-adhesion U-shaped culture chamber; and the U-shaped culture well is a low-adhesion U-shaped culture well.

In a second aspect, the present disclosure provides a vascularized organoid spheroid model, wherein the vascularized organoid spheroid model is constructed by the above method. The vascularized organoid spheroid model has a spheroid structure, with a plurality of organoids aggregated and growing inside. Microvessels grow divergently from the inside to the outside of the vascularized organoid spheroid model. The vascularized organoid spheroid model further includes an organoid microenvironment composed of fibroblast structures and microvessels.

In some embodiments, the vascularized organoid spheroid model has cell proliferation, wherein the extent of cell proliferation increases in a gradient from inside to outside, and the oxygen content of the vascularized organoid spheroid model gradually increases from inside to outside.

In some embodiments, the diameter of the vascularized organoid spheroid model ranges from 250 µm to 800 µm. Preferably, the diameter of the vascularized organoid spheroid model ranges from 300 µm to 600 µm.

In a third aspect, the present disclosure provides use of the above vascularized organoid spheroid model in testing, wherein the testing includes any one of drug screening, drug sensitivity testing, drug toxicity testing or non-drug substance toxicity testing.

In some embodiments, the use includes at least: adding a drug to be tested to the culture chamber or the culture well containing the vascularized organoid spheroid model; performing culture for 3 to 5 days after placement in a culture environment, and detecting a change in an indicator of the vascularized organoid spheroid model. The change in the indicator includes at least one of a change in shape, a change in size, a change in viability, a change in vascular morphology, a change in protein molecular expression or a change in distribution of the vascularized organoid spheroid model.

In other embodiments, the use includes at least: adding a drug to be tested to a culture chamber of a drug-sensitive chip or a culture well of a drug-sensitive chip containing the vascularized organoid spheroid model; performing dynamic culture for 3 to 5 days after placement in a culture environment; and detecting a change in an indicator of the vascularized organoid spheroid model. The change in the indicator includes at least one of a change in shape, a change in size, a change in viability, a change in vascular morphology, a change in protein molecular expression or a change in distribution of the vascularized organoid spheroid model.

In a fourth aspect, the present disclosure provides a drug testing method, including: S100: preparing or obtaining an organoid, digesting the organoid to obtain organoid cells, and obtaining fibroblasts and vascular endothelial cells; S200: mixing the organoid cells, the fibroblasts and the vascular endothelial cells, and resuspending them with a culture medium to obtain an organoid multicellular suspension; S300: inoculating the organoid multicellular suspension into a culture chamber or a culture well, and performing spheroid culture, allowing self-assembly to form a vascularized organoid spheroid model; and S400: adding a drug to be tested to the culture chamber or the culture well containing the vascularized organoid spheroid model, performing culture after placement in a culture environment, and then detecting a change in an indicator of the vascularized organoid spheroid model. For the selection of organoid and specific parameters, reference may be made to the contents of the above three aspects, which will not be repeated.

In a fifth aspect, the present disclosure provides a culture medium composition, including a first culture medium for culturing organoid cells and a second culture medium for culturing vascular endothelial cells, wherein the mass ratio of the first culture medium to the second culture medium is 1: (0.5-2).

In some preferred embodiments, the mass ratio of the first culture medium to the second culture medium is 1: (0.8-1.2).

For the organoid cells, reference may be made to the contents of the first to third aspects, which will not be repeated.

Compared with the prior art, the technical solution of the present disclosure has at least the following technical effects: the present disclosure constructs a vascularized organoid spheroid model by co-culturing organoid cells, fibroblasts and vascular endothelial cells. During the culture process, a U-shaped bottom culture chamber or a U-shaped bottom culture well is used for multi-stage spheroid culture to form a dense spheroid structure. At the same time, by regulating the cell number ratio of the three types of cells within an appropriate range, the present disclosure ensures that the intercellular connections in the prepared vascularized organoid spheroid model are tighter and the biomimetic fidelity is better, and it can fully represent the original tissue. Compared with the models in the prior art, it is more suitable for detection, especially for the detection of drugs related to anti-tumor angiogenesis.

### BRIEF DESCRIPTION OF THE DRAWINGS

In order to more clearly illustrate the technical solutions in the examples of the present disclosure or the prior art, a brief introduction will be given below to the drawings that need to be used in the description of the examples or the prior art. Obviously, the drawings in the following description are only some examples of the present disclosure. For those skilled in the art, other drawings can be obtained based on these drawings without creative work.
FIG. 1 is a test image of the vascularized lung cancer organoid spheroid model prepared in Example 1 of the present disclosure.
FIG. 2 is a comparative test image comparing the two cases of digesting a lung cancer organoid into single cells and not digesting it into single cells during the preparation of the vascularized lung cancer organoid spheroid model in Example 1 of the present disclosure.
FIG. 3 is a comparative test image of changing the cell number ratio between cells during the preparation of the vascularized lung cancer organoid spheroid model in Example 1 of the present disclosure.
FIG. 4 is an observation image of the vascularized lung cancer organoid spheroid model prepared in Example 1 of the present disclosure during the drug sensitivity test.
FIG. 5 is a diagram showing the results of testing the lysates of the three groups of experiments in FIG. 4 using a chemiluminescence microplate reader.
FIG. 6 is an observation image of the vascularized lung cancer organoid spheroid model prepared in Example 2 of the present disclosure during the drug sensitivity test.
FIG. 7 is a diagram showing the results of testing the lysates in FIG. 6 using a chemiluminescence microplate reader.
FIG. 8 is an observation image of the vascularized colorectal cancer organoid spheroid model prepared in Example 3 of the present disclosure during the drug sensitivity test.
FIG. 9 is a diagram showing the results of testing the lysates in FIG. 8 using a chemiluminescence microplate reader.
FIG. 10 is an observation image of the vascularized pancreatic cancer organoid spheroid model prepared in Example 4 of the present disclosure during the drug sensitivity test.
FIG. 11 is a diagram showing the results of testing the lysates in FIG. 10 using a chemiluminescence microplate reader.
FIG. 12 is an observation image of the vascularized liver organoid spheroid model prepared in Example 5 of the present disclosure during the hepatotoxicity test.
FIG. 13 is a diagram showing the results of testing the lysates of the three groups of experiments in FIG. 12 using a chemiluminescence microplate reader.
FIG. 14 is a schematic structural diagram of the drug-sensitive chip used in the present disclosure.

### DETAILED DESCRIPTION

In order to make the objectives, technical solutions and advantages of the present disclosure clearer, the present disclosure will be further described in detail below with reference to the accompanying drawings and examples. It should be understood that the specific examples described herein are only used to explain the present disclosure and are not used to limit the present disclosure.

### Method for Constructing a Vascularized Lung Cancer Organoid Spheroid Model

S100: Preparing or obtaining a lung cancer organoid, digesting the lung cancer organoid to obtain lung cancer organoid cells, and obtaining fibroblasts and vascular endothelial cells.

The lung cancer organoid cells are selected from a lung cancer organoid extracted from tissue or a lung cancer organoid differentiated from iPS stem cells. The organoid can be either a primary lung cancer organoid or a passaged lung cancer organoid.

Preferably, the lung cancer organoid cells are selected from lung cancer organoid cells obtained by digesting the primary lung cancer organoid into single cells or lung cancer organoid cells obtained by digesting the passaged lung cancer organoid into single cells.

S200: Mixing the lung cancer organoid cells, the fibroblasts and the vascular endothelial cells, and resuspending them with a culture medium to obtain a lung cancer organoid multicellular suspension.

The cell number ratio of the lung cancer organoid cells, the fibroblasts and the vascular endothelial cells is 1: (0.1-1): (0.05-1).

Preferably, the fibroblasts include lung cancer-associated fibroblasts, and the cell number ratio of the lung cancer organoid cells, the lung cancer-associated fibroblasts and the vascular endothelial cells is 1: (0.4-0.8): (0.1-0.5).

The culture medium is a mixed culture medium, and the mixed culture medium includes at least a first culture medium for culturing the lung cancer organoid and a second culture medium for culturing the vascular endothelial cells. The mass ratio of the first culture medium to the second culture medium is 1: (0.5-2). Preferably, the mass ratio of the first culture medium to the second culture medium is 1: (0.8-1.2).

S300: Inoculating the lung cancer organoid multicellular suspension into a culture chamber or a culture well, and performing spheroid culture, allowing self-assembly to form a vascularized lung cancer organoid spheroid model.

Specifically, the lung cancer organoid multicellular suspension is inoculated into a culture chamber with a U-shaped bottom or a culture well with a U-shaped bottom for spheroid culture. The spheroid culture includes a first-stage spheroid culture and a second-stage spheroid culture performed sequentially. The first-stage spheroid culture includes: inoculating the lung cancer organoid multicellular suspension into the culture chamber with a U-shaped bottom or the culture well with a U-shaped bottom, centrifuging at 950 rpm to 1,050 rpm for 5 min to 6 min, and performing culture after placement in a culture environment of 37°C and 5% CO₂. The culture time of the first-stage spheroid culture is 1 to 5 days. The above centrifugation step is only an illustrative example, and other methods can also be used to aggregate cells, such as group modification on a chip. After the first-stage spheroid culture is completed, a lung cancer organoid spheroid model intermediate with clear boundaries and microvessels is obtained, and then the second-stage spheroid culture is performed.

In some embodiments, the second-stage spheroid culture includes: discarding the original culture medium in the culture chamber or the culture well, adding a cell culture scaffold material such as hydrogel or matrigel, adding the mixed culture medium after gelation, and performing culture for 1 to 2 days after placement in a culture environment of 37°C and 5% CO₂.

In other embodiments, the second-stage spheroid culture includes: discarding the original culture medium in the culture chamber or the culture well, adding a cell culture scaffold material such as hydrogel or matrigel, transferring a resulting mixture to a culture chamber of a drug-sensitive chip or a culture well of a drug-sensitive chip, adding the mixed culture medium after gelation, and performing dynamic culture for 1 to 2 days after placement in a culture environment of 37°C and 5% CO₂, wherein the culture chambers of the drug-sensitive chip and the culture wells of the drug-sensitive chip both have U-shaped bottoms and are interconnected.

The dynamic culture includes: performing flow perfusion culture at a speed of rocking 3° to 10° every 15 min to 120 min in a culture environment. Exemplarily, flow perfusion culture is performed at a rocking angle of 3°/120 min in a culture environment of 37°C and 5% CO₂; or flow perfusion culture is performed at a rocking angle of 7°/30 min in a culture environment of 37°C and 5% CO₂; or flow perfusion culture is performed at a rocking angle of 7°/15 min in a culture environment of 37°C and 5% CO₂. The dynamic culture is the same in the following illustrative examples and will not be repeated.

### Method for Constructing a Vascularized Colorectal Cancer Organoid Spheroid Model

S100: Preparing or obtaining an organoid, wherein the organoid is a primary colorectal cancer organoid or a passaged colorectal cancer organoid, digesting the primary colorectal cancer organoid or the passaged colorectal cancer organoid into single cells to obtain colorectal cancer organoid cells, and obtaining colorectal cancer-associated fibroblasts and vascular endothelial cells.

S200: Mixing the colorectal cancer organoid cells, the colorectal cancer-associated fibroblasts and the vascular endothelial cells, and resuspending them with a culture medium to obtain a colorectal cancer organoid multicellular suspension.

The cell number ratio of the colorectal cancer organoid cells, the colorectal cancer-associated fibroblasts and the vascular endothelial cells is 1: (0.1-0.8): (0.2-0.6).

The culture medium is a mixed culture medium, and the mixed culture medium includes at least a first culture medium for culturing the colorectal cancer organoid cells and a second culture medium for culturing the vascular endothelial cells. In the mixed culture medium, the mass ratio of the first culture medium to the second culture medium is 1: (0.5-2).

S300: Inoculating the colorectal cancer organoid multicellular suspension into a culture chamber or a culture well, and performing spheroid culture, allowing self-assembly to form a vascularized colorectal cancer organoid spheroid model.

Specifically, the colorectal cancer organoid multicellular suspension is inoculated into a culture chamber with a U-shaped bottom or a culture well with a U-shaped bottom for spheroid culture. The spheroid culture includes a first-stage spheroid culture and a second-stage spheroid culture performed sequentially. In some embodiments, the first-stage spheroid culture includes: inoculating the colorectal cancer organoid multicellular suspension into the culture chamber with a U-shaped bottom or the culture well with a U-shaped bottom, centrifuging at 800 rpm to 1,200 rpm for 5 min to 10 min, and performing culture after placement in a culture environment of 37°C and 5% CO₂. The culture time of the first-stage spheroid culture is 1 to 5 days. After the first-stage spheroid culture is completed, a colorectal cancer organoid spheroid model intermediate with clear boundaries and microvessels is obtained, and then the second-stage spheroid culture is performed.

In some embodiments, the second-stage spheroid culture includes: discarding the original culture medium in the culture chamber or the culture well, adding a cell culture scaffold material such as hydrogel or matrigel, adding the mixed culture medium after gelation, and continuing to perform culture for 1 to 2 days after placement in a culture environment of 37°C and 5% CO₂.

In other embodiments, the second-stage spheroid culture includes: discarding the original culture medium in the culture chamber or the culture well, adding a cell culture scaffold material such as hydrogel or matrigel, transferring a resulting mixture to a culture chamber of a drug-sensitive chip or a culture well of a drug-sensitive chip, adding the culture medium after gelation, and performing dynamic culture for 1 to 2 days after placement in a culture environment, wherein the culture chambers of the drug-sensitive chip or the culture wells of the drug-sensitive chip have U-shaped bottoms and are interconnected.

### Method for Constructing a Vascularized Pancreatic Cancer Organoid Spheroid Model

S100: Preparing or obtaining an organoid, wherein the organoid is a primary pancreatic cancer organoid or a passaged pancreatic cancer organoid, digesting the primary pancreatic cancer organoid or the passaged pancreatic cancer organoid into single cells to obtain pancreatic cancer organoid cells, and obtaining pancreatic cancer-associated fibroblasts and vascular endothelial cells.

S200: Mixing the pancreatic cancer organoid cells, the pancreatic cancer-associated fibroblasts and the vascular endothelial cells, and resuspending them with a culture medium to obtain a pancreatic cancer organoid multicellular suspension.

The cell number ratio of the pancreatic cancer organoid cells, the pancreatic cancer-associated fibroblasts and the vascular endothelial cells is 1: (0.2-0.7): (0.2-0.7).

The culture medium is a mixed culture medium, and the mixed culture medium includes at least a first culture medium for culturing the pancreatic cancer organoid cells and a second culture medium for culturing the vascular endothelial cells. In the mixed culture medium, the mass ratio of the first culture medium to the second culture medium is 1: (0.5-2).

S300: Inoculating the pancreatic cancer organoid multicellular suspension into a culture chamber or a culture well, and performing spheroid culture, allowing self-assembly to form a vascularized pancreatic cancer organoid spheroid model.

Specifically, the pancreatic cancer organoid multicellular suspension is inoculated into a culture chamber with a U-shaped bottom or a culture well with a U-shaped bottom for spheroid culture. The spheroid culture includes a first-stage spheroid culture and a second-stage spheroid culture performed sequentially. In some embodiments, the first-stage spheroid culture includes: inoculating the pancreatic cancer organoid multicellular suspension into the culture chamber with a U-shaped bottom or the culture well with a U-shaped bottom, centrifuging at 800 rpm to 1,200 rpm for 5 min to 10 min, and performing culture after placement in a culture environment of 37°C and 5% CO₂. The culture time of the first-stage spheroid culture is 1 to 5 days. A pancreatic cancer organoid spheroid model intermediate with clear boundaries and microvessels is obtained, and then the second-stage spheroid culture is performed.

In some embodiments, the second-stage spheroid culture includes: discarding the original culture medium in the culture chamber or the culture well, adding a cell culture scaffold material such as hydrogel or matrigel, adding the mixed culture medium after gelation, and performing culture for 1 to 2 days after placement in a culture environment of 37°C and 5% CO₂.

In other embodiments, the second-stage spheroid culture includes: discarding the original culture medium in the culture chamber or the culture well, adding a cell culture scaffold material such as hydrogel or matrigel, transferring a resulting mixture to a culture chamber of a drug-sensitive chip or a culture well of a drug-sensitive chip, adding the mixed culture medium after gelation, and performing dynamic culture for 1 to 2 days after placement in a culture environment of 37°C and 5% CO₂, wherein the culture chambers of the drug-sensitive chip or the culture wells of the drug-sensitive chip have U-shaped bottoms and are interconnected.

### Method for constructing a vascularized liver organoid spheroid model

S100: Preparing or obtaining an organoid, wherein the organoid is a primary liver organoid or a passaged liver organoid, digesting the primary liver organoid or the passaged liver organoid into single cells to obtain liver organoid cells, and obtaining liver-associated fibroblasts and vascular endothelial cells.

S200: Mixing the liver organoid cells, the liver-associated fibroblasts and the vascular endothelial cells, and resuspending them with a culture medium to obtain a liver organoid multicellular suspension.

The cell number ratio of the liver organoid cells, the liver-associated fibroblasts and the vascular endothelial cells is 1: (1-2): (1-2).

The culture medium is a mixed culture medium, and the mixed culture medium includes at least a first culture medium for culturing the liver organoid cells and a second culture medium for culturing the vascular endothelial cells. The mass ratio of the first culture medium to the second culture medium is 1: (0.5-2).

S300: Inoculating the liver organoid multicellular suspension into a culture chamber or a culture well, and performing spheroid culture, allowing self-assembly to form a vascularized liver organoid spheroid model.

Specifically, the liver organoid multicellular suspension is inoculated into a culture chamber with a U-shaped bottom or a culture well with a U-shaped bottom for spheroid culture. The spheroid culture includes a first-stage spheroid culture and a second-stage spheroid culture performed sequentially. In some embodiments, the first-stage spheroid culture includes: inoculating the liver organoid multicellular suspension into the culture chamber with a U-shaped bottom or the culture well with a U-shaped bottom, centrifuging at 800 rpm to 1,200 rpm for 5 min to 10 min, and performing culture for 1 to 5 days after placement in a culture environment, to obtain a liver organoid spheroid model intermediate with clear boundaries and microvessels, enabling the second-stage spheroid culture to be performed subsequently.

In some embodiments, the second-stage spheroid culture includes: discarding the original culture medium in the culture chamber or the culture well, adding a cell culture scaffold material such as hydrogel or matrigel, adding the culture medium after gelation, and continuing to perform culture for 1 to 2 days after placement in a culture environment.

In other embodiments, the second-stage spheroid culture includes: discarding the original culture medium in the culture chamber or the culture well, adding a cell culture scaffold material such as hydrogel or matrigel, transferring a resulting mixture to a culture chamber of a drug-sensitive chip or a culture well of a drug-sensitive chip, adding the culture medium after gelation, and performing dynamic culture for 1 to 2 days after placement in a culture environment, wherein the culture chambers of the drug-sensitive chip or the culture wells of the drug-sensitive chip have U-shaped bottoms and are interconnected.

### Illustrative Example 1

### Preparation of Vascularized Lung Cancer Organoid Spheroid Model (Formation of Vascularized Lung Cancer Organoid Spheroid Model on U-shaped Well Plate)

S100: Lung cancer organoid cells, fibroblasts and vascular endothelial cells were obtained.

Step 1: A primary lung cancer organoid or a passaged lung cancer organoid was selected. Pre-chilled PBS was added to a culture well plate, PBS was added to each well, and the contents were collected into a centrifuge tube. The tube was refrigerated at 4°C to 6°C for 15 min to 20 min and then centrifuged. The supernatant was removed to obtain a lung cancer organoid pellet.

Step 2: Tryple E was added for enzymatic digestion for 5 min to 10 min. After digestion into single cells, the cells were centrifuged. The supernatant was removed, PBS was added, and the mixture was centrifuged again to remove excess enzyme solution. A pellet was obtained, which was resuspended in a mixed culture medium. Cell counting was performed to obtain the cell concentration of the lung cancer organoid suspension.

In the mixed culture medium, the mass ratio of the first culture medium to the second culture medium was 1: (0.5-2).

Step 3: Lung cancer-associated fibroblasts were taken, digested into single cells, resuspended in the above mixed culture medium, and counted.

Step 4: Vascular endothelial cells were taken, digested into single cells, resuspended in the above mixed culture medium, and counted.

S200: The lung cancer organoid cells, fibroblasts and vascular endothelial cells were mixed and resuspended with a culture medium to obtain a lung cancer organoid multicellular suspension.

Step 5: The lung cancer organoid cells, lung cancer-associated fibroblasts and vascular endothelial cells were mixed according to a cell number ratio of 1: (0.4-0.8): (0.1-0.5), and resuspended with the above mixed culture medium to obtain a lung cancer organoid multicellular suspension.

S300: The lung cancer organoid multicellular suspension was inoculated into a culture chamber or a culture well, spheroid culture was performed, and the vascularized lung cancer organoid spheroid model was formed via self-assembly.

Step 6: The lung cancer organoid multicellular suspension was added to a low-adhesion U-shaped bottom culture well and centrifuged at 950 rpm to 1,050 rpm for 5 min to 6 min.

Step 7: The plate was placed into a culture environment of 37°C and 5% CO₂ for first-stage spheroid culture.

Step 8: After culturing for 1 to 5 days, the original culture medium in the well plate was aspirated. A cell culture scaffold material such as hydrogel or matrigel was added. After gelation, the above mixed culture medium was added, and the plate was placed into a culture environment of 37°C and 5% CO₂ for second-stage spheroid culture. The culture time of the second-stage spheroid culture was 1 to 2 days.

### Drug testing of Illustrative Example 1

(1) A drug-containing culture medium at an appropriate concentration was prepared.
(2) The vascularized lung cancer organoid spheroid model formed by the above culture was transferred to a 96-well plate.
(3) The growth state of the vascularized lung cancer organoid spheroid model was observed and recorded. The original culture medium in the 96-well plate was aspirated, 0.01 µM to 100 µM of drug-containing culture medium was added to each well, and the plate was placed into an incubator at 37°C with 5% CO₂ for culture.
(4) On Day 3, the growth state of the vascularized lung cancer organoid spheroid model was observed and recorded.
(5) On Day 5, the growth state of the vascularized lung cancer organoid spheroid model was observed and recorded, and viability detection was performed using a CTG detection kit.
   80 µL to 120 µL of CTG detection reagent was added to each well. The plate was shaken on a shaker for 5 to 10 min, and incubated at room temperature for 20 to 30 min.
(6) After incubation, detection was performed using a chemiluminescence microplate reader.

### Illustrative Example 2

### Preparation of Vascularized Lung Cancer Organoid Spheroid Model (Formation of Vascularized Lung Cancer Organoid Spheroid Model on Drug-sensitive Chip)

S100: Lung cancer organoid cells, fibroblasts and vascular endothelial cells were obtained.

Step 1: A primary lung cancer organoid or a passaged lung cancer organoid was selected. Pre-chilled PBS was added to a culture well plate, 1 mL to 2 mL of PBS was added to each well, and the contents were collected into a centrifuge tube. The tube was refrigerated at 4°C to 6°C for 15 min to 20 min, and then centrifuged. The supernatant was removed to obtain a lung cancer organoid pellet.

Step 2: Tryple E was added for enzymatic digestion for 5 min to 10 min. After digestion into single cells, the cells were centrifuged. The supernatant was removed, and PBS was added, and the mixture was centrifuged again to remove excess enzyme solution. A pellet was obtained, which was resuspended in a mixed culture medium. Cell counting was performed to obtain the cell concentration of the lung cancer organoid suspension.

The mixed culture medium in Illustrative Example 2 was the same as that in Illustrative Example 1, and will not be repeated.

Step 3: Lung cancer-associated fibroblasts were taken, digested into single cells, resuspended in the above mixed culture medium, and counted.

Step 4: Vascular endothelial cells were taken, digested into single cells, resuspended in the above mixed culture medium, and counted.

S200: The lung cancer organoid cells, fibroblasts and vascular endothelial cells were mixed and resuspended with a culture medium to obtain a lung cancer organoid multicellular suspension.

Step 5: The lung cancer organoid cells, the lung cancer-associated fibroblasts and the vascular endothelial cells were mixed according to a cell number ratio of 1: (0.4-0.8): (0.1-0.5), and resuspended with the above mixed culture medium to obtain a lung cancer organoid multicellular suspension.

S300: The lung cancer organoid multicellular suspension was inoculated into a culture chamber or a culture well, spheroid culture was performed, and the vascularized lung cancer organoid spheroid model was formed via self-assembly.

Step 6: The lung cancer organoid multicellular suspension was added to a low-adhesion U-shaped bottom culture chamber, and centrifuged at 950 rpm to 1,050 rpm for 5 min to 6 min.

Step 7: The plate was placed into a culture environment of 37°C and 5% CO₂ for first-stage spheroid culture.

Step 8: After culturing for 1 to 5 days, the original culture medium in the well plate was aspirated. A cell culture scaffold material such as hydrogel or matrigel was added. The spheroid was transferred to the middle well of a drug-sensitive chip. After gelation, 25 µL to 35 µL of mixed culture medium was added to the middle well of the drug-sensitive chip, and 45 µL to 55 µL of mixed culture medium was added to each of the two side wells of the drug-sensitive chip. The drug-sensitive chip was placed in a rocking perfusion bioreactor, and flow perfusion culture was performed for 1 to 2 days at a speed of rocking 3° to 10° every 15 min to 120 min in a culture environment of 37°C and 5% CO₂.

### Drug testing of Illustrative Example 2

(1) A drug-containing culture medium at an appropriate concentration was prepared.
(2) The growth state of a vascularized lung cancer organoid spheroid model in a culture chamber of a drug-sensitive chip was observed and recorded. The original culture medium in the culture chamber was aspirated.
(3) 45 µL to 55 µL of drug-containing culture medium was added to each of the two side reservoir chambers of the drug-sensitive chip (corresponding to the two side wells of the drug-sensitive chip), and 25 µL to 35 µL of drug-containing culture medium was added to the middle culture chamber (corresponding to the middle well of the drug-sensitive chip).
(4) The drug-loaded drug-sensitive chip was placed in a rocking perfusion bioreactor, and the condition was set to 3°/120 min. The rocking perfusion bioreactor was then placed into an incubator at 37°C with 5% CO₂ for flow perfusion culture.
(5) On Day 3, the growth state of the vascularized lung cancer organoid spheroid model was observed and recorded, and 25 µL to 35 µL of drug-containing culture medium was further added to the middle culture chamber.
(6) On Day 5, the growth state of the vascularized lung cancer organoid spheroid model was observed and recorded, and viability detection was performed using a CTG detection kit.
(7) All drug-containing culture medium was discarded from the two side reservoir chambers, and 20 µL to 30 µL of CTG dilution solution (1:1 dilution of CTG reagent stock solution and mixed culture medium) was added.
(8) The drug-containing culture medium in the middle culture chamber was not discarded; 45 µL to 55 µL of CTG stock solution was directly added and mixed by pipetting.
(9) The chip was shaken on a shaker for 5 min to 10 min and incubated at room temperature for 25 min to 30 min.
(10) All the lysate was taken out and put into a 96-well plate that was completely opaque on all sides and bottom, and detection was performed using a chemiluminescence microplate reader.

The culture time during the drug testing may be set to be shorter or longer according to the action and metabolic rate of the drug.

It should be noted that the construction of other models in the present disclosure, such as the vascularized colorectal cancer organoid spheroid model, the vascularized pancreatic cancer organoid spheroid model and other non-tumor organoid models, may follow the exemplary methods described above. The difference lies in the selection of organoid cell types and fibroblast types during the mixing process of organoid cells, fibroblasts and vascular endothelial cells, as well as the corresponding cell number ratios. Other aspects may follow the exemplary construction methods described above. Furthermore, the drug testing of other models may follow the test steps of Illustrative Example 1 or Illustrative Example 2, with the only difference being the type and the concentration of the drug in the drug-containing culture medium.

Hereinafter, examples and comparative examples are provided to illustrate the implementations of the present disclosure in more detail. The reagents and instruments used in the following examples and comparative examples are all commercially available.

Optionally, the lung cancer organoid culture kit is purchased from Jiangsu AVATARGET Biotechnology Co., Ltd., catalog number KLU0101.

The colorectal cancer organoid culture kit is purchased from bioGenous Biotechnology (Suzhou) Co., Ltd., catalog number K2103-CR; the pancreatic cancer organoid culture kit is purchased from Jiangsu AVATARGET Biotechnology Co., Ltd., catalog number KPA0101; the liver organoid culture kit is purchased from Jiangsu AVATARGET Biotechnology Co., Ltd., catalog number KLV0101.

The non-small cell lung cancer organoid culture medium (i.e., the first culture medium) is purchased from Jiangsu AVATARGET Biotechnology Co., Ltd., catalog number KCJ-6-1.

Human primary lung cancer-associated fibroblasts are purchased from Hunan Meisen Biotechnology Co., Ltd., catalog number CTCC-258-Hum.

Human umbilical vein endothelial cells HUVEC are purchased from the Cell Resource Center, Peking Union Medical College, catalog number 1101HUM-PUMC000437.

The colorectal cancer organoid culture medium is purchased from Jiangsu AVATARGET Biotechnology Co., Ltd., catalog number KCJ-2; the pancreatic cancer organoid culture medium is purchased from Jiangsu AVATARGET Biotechnology Co., Ltd., catalog number KCJ-3.

The low-adhesion U-shaped bottom 96-well plate is purchased from Shanghai Nuo Ning Biotechnology Co., Ltd., catalog number NBH1296.

### Example 1

### (I) Preparation of Vascularized Lung Cancer Organoid Spheroid Model (Formation of Vascularized Lung Cancer Organoid Spheroid Model on U-shaped Well Plate)

Step 1: A primary lung cancer organoid (i.e., the lung cancer organoid culture kit with catalog number KLU0101, same as in Example 2) was selected. Pre-chilled PBS was added to a culture well plate, 1 mL of PBS was added to each well, and the contents were collected into a centrifuge tube. The tube was refrigerated at 4°C for 15 min and then centrifuged (1,000 rpm, 5 min). The supernatant was removed to obtain a lung cancer organoid pellet.

Step 2: Tryple E was added for enzymatic digestion for 8 min. After digestion into single cells, the cells were centrifuged (1,000 rpm, 5 min). The supernatant was removed, PBS was added, and the mixture was centrifuged again (1,000 rpm, 5 min) to remove excess enzyme solution. A pellet was obtained, which was resuspended in a mixed culture medium. Cell counting was performed to obtain the cell concentration of the lung cancer organoid suspension.

In the mixed culture medium, the mass ratio of the first culture medium to the second culture medium was 1:0.8 (same as in Example 2, not repeated).

Step 3: Human primary lung cancer-associated fibroblasts (i.e., lung cancer-associated fibroblasts, the same applies below) were taken, digested into single cells, resuspended in the above mixed culture medium, and counted.

Step 4: Human umbilical vein endothelial cells HUVEC (i.e., vascular endothelial cells, the same applies below) were taken, digested into single cells, resuspended in the above mixed culture medium, and counted.

Step 5: Lung cancer organoid cells, human primary lung cancer-associated fibroblasts, and human umbilical vein endothelial cells HUVEC were mixed according to a cell number ratio of 1:0.75:0.25 (the total number of cells per well was 5,000, the number of lung cancer organoid cells was 2,500/well, the number of human primary lung cancer-associated fibroblasts was 1,875/well, the number of human umbilical vein endothelial cells was 625/well) to obtain a lung cancer organoid multicellular suspension.

Step 6: The above lung cancer organoid multicellular suspension was added to a low-adhesion U-shaped bottom 96-well plate and centrifuged at 1,000 rpm for 5 min.

Step 7: The plate was placed into an incubator at 37°C with 5% CO₂ for first-stage spheroid culture.

Step 8: After culturing for 3 days, the original culture medium in the well plate was aspirated, 5 µL of hydrogel was added. After gelation, 100 µL of the above mixed culture medium was added, and the plate was placed into an incubator at 37°C with 5% CO₂ for second-stage spheroid culture. The culture time of the second-stage spheroid culture was 2 days.

The diameter of the vascularized lung cancer organoid spheroid model prepared in Example 1 was 400 µm.

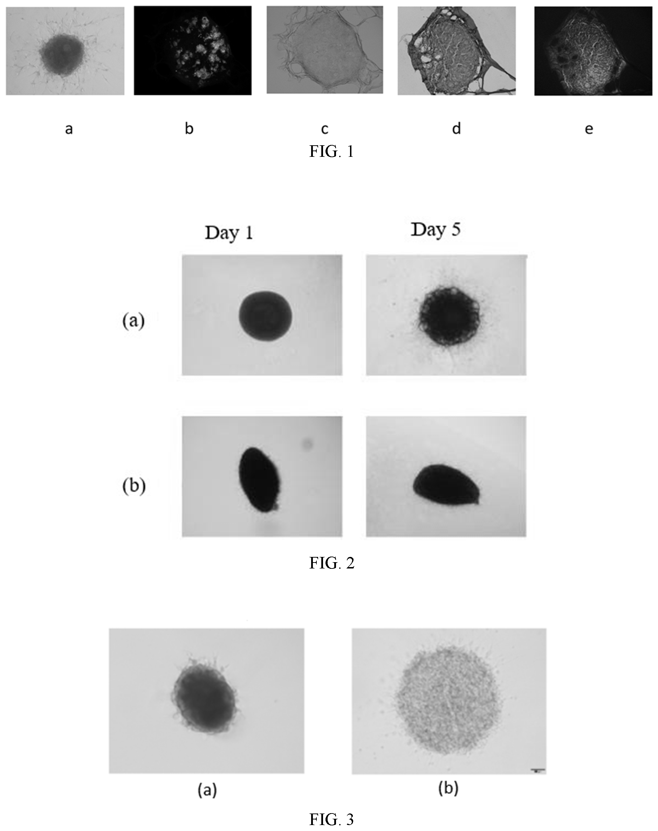

FIG. 1 is a test image of the vascularized lung cancer organoid spheroid model prepared in Example 1 of the present disclosure.

FIG. 1a shows a bright-field image of the vascularized lung cancer organoid spheroid model. It can be seen that the vascularized lung cancer organoid spheroid model prepared in Example 1 has clear boundaries and is grown with a large-area of microvessels presenting a complex network structure. In FIG. 1b, a green cell tracker is used to label the lung cancer organoid. From the distribution diagram of the lung cancer organoid, it can be seen that the lung cancer organoid grows uniformly in a spherical shape. FIG. 1c shows an unstained paraffin section of the lung cancer organoid. It can be seen that the spheroid cells grow densely, and the peripheral endothelial cells grow in a tubular shape. FIG. 1d shows a paraffin section of the lung cancer organoid stained with hematoxylin and eosin. It can be seen that lung cancer organoid cells, lung cancer-associated fibroblasts and vascular endothelial cells grow in a fused manner, with the three types of cells promoting each other to form a spheroid. FIG. 1e shows a fluorescence image of microvessels generated in the lung cancer organoid multicellular spheroid. A green cell tracker is used to label the lung cancer organoid. The lung cancer organoid multicellular spheroid is subjected to frozen sectioning. The green fluorescence indicates that the organoid is distributed in the spheroid and grows in fusion with endothelial cells and fibroblasts. From the test images in FIG. 1, it can be seen that the vascularized lung cancer organoid spheroid model prepared in Example 1 of the present disclosure has good biomimetic fidelity.

In FIG. 2, two cases during the preparation of the vascularized lung cancer organoid spheroid model in Example 1 of the present disclosure are compared: digesting a lung cancer organoid into single cells versus not digesting it into single cells. FIG. 2(a) shows the state on Day 1 and Day 5 of culture when the lung cancer organoid was digested into single cells and then mixed with other cells in the process of obtaining lung cancer organoid cells. FIG. 2(b) shows the state on Day 1 and Day 5 of culture when the lung cancer organoid was not digested into single cells but directly mixed with other cells in the process of obtaining lung cancer organoid cells. From the comparison photos in FIG. 2, it can be seen that digesting the lung cancer organoid into single cells and then mixing with other cells to form multicellular spheroids is more likely to form spheroids. At the same time, after 5 days of spheroid growth, it can be seen that after the organoid was digested into single cells and then formed into multicellular spheroids, the microvessels of the model grow more abundantly.

In FIG. 3, the cell number ratio of lung cancer organoid cells, lung cancer-associated fibroblasts and vascular endothelial cells during the preparation of the vascularized lung cancer organoid spheroid model in Example 1 of the present disclosure are further compared. In FIG. 3(a), the cell number ratio of lung cancer organoid cells, lung cancer-associated fibroblasts and vascular endothelial cells was 4:3:1, showing the bright-field image after 3 days of culture following mixing. In FIG. 3(b), the cell number ratio of lung cancer organoid cells, lung cancer-associated fibroblasts and vascular endothelial cells was 30:1:2, showing the bright-field image after 3 days of culture following mixing. From FIG. 3, it can be seen that a suitable cell number ratio of lung cancer organoid cells, lung cancer-associated fibroblasts and vascular endothelial cells is conducive to spheroid formation and also more conducive to the construction of a tumor microenvironment, allowing for the growth of a lung cancer organoid model with a complex network of microvessels and better biomimetic fidelity.

### (II) Drug testing

(1) Drug-containing culture media at appropriate concentrations were prepared: Cisplatin + Paclitaxel (Cisplatin 6 µM, Paclitaxel 4 µM), Pemetrexed (20 µM).
(2) The vascularized lung cancer organoid spheroid model formed by culture in Example 1 was transferred to a 96-well plate.
(3) The growth state of the vascularized lung cancer organoid spheroid model was observed and recorded. The original culture medium in the 96-well plate was aspirated, 100 µL of drug-containing culture medium was added to each well, and the plate was placed into an incubator at 37°C with 5% CO₂ for culture.
(4) On Day 3, the growth state of the vascularized lung cancer organoid spheroid model was observed and recorded.
(5) On Day 5, the growth state of the vascularized lung cancer organoid spheroid model was observed and recorded, and viability detection was performed using a CTG detection kit.
   100 µL of CTG detection reagent was added to each well. The plate was shaken on a shaker for 5 min, and incubated at room temperature for 25 min.
(6) After incubation, detection was performed using a chemiluminescence microplate reader.

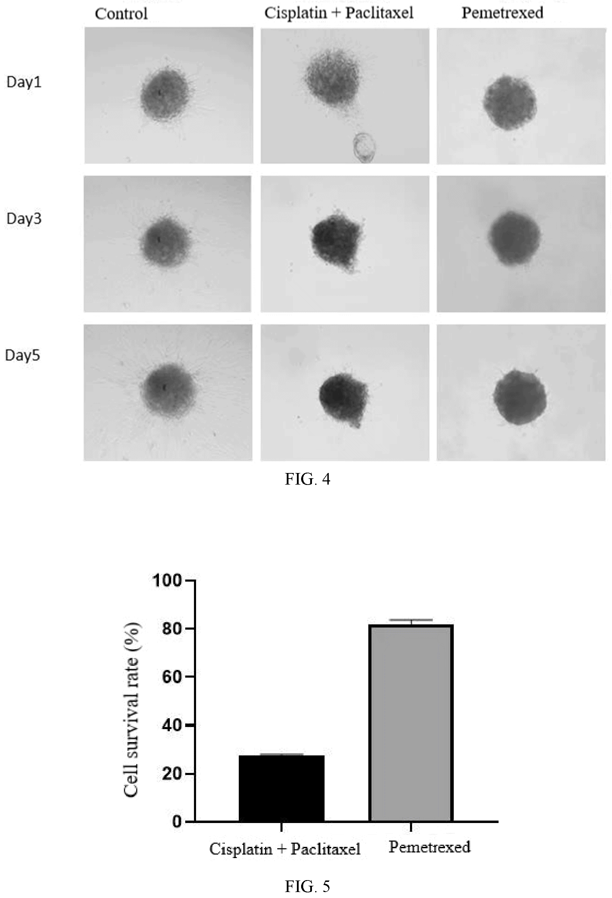

FIG. 4 shows the state of the vascularized lung cancer organoid spheroid model prepared in Example 1 of the present disclosure on Day 3 and Day 5 of culture after adding the drug-containing culture medium during the drug sensitivity test. The culture medium in the control group was blank culture medium, and the blank culture medium is prepared by adding 1% DMSO to the above mixed culture medium.

FIG. 5 is a diagram showing the results of testing the lysates of the three groups of experiments in FIG. 4 using a chemiluminescence microplate reader.

Combining FIG. 4 and FIG. 5, it can be seen that both Cisplatin + Paclitaxel (a combination drug) and Pemetrexed have a certain vascular inhibitory effect on the vascularized lung cancer organoid spheroid model constructed in Example 1 of the present disclosure, and the combination drug of Cisplatin + Paclitaxel has a stronger sensitivity to this lung cancer organoid spheroid.

### Example 2

### (I) Preparation of Vascularized Lung Cancer Organoid Spheroid Model (Formation of Vascularized Lung Cancer Organoid Spheroid Model on Drug-sensitive Chip)

Step 1: A primary lung cancer organoid was selected. Pre-chilled PBS was added to a culture well plate, 1 mL of PBS was added to each well, and the contents were collected into a centrifuge tube. The tube was refrigerated at 4°C for 15 min and then centrifuged (1,000 rpm, 5 min). The supernatant was removed to obtain a lung cancer organoid pellet.

Step 2: Tryple E was added for enzymatic digestion for 8 min. After digestion into single cells, the cells were centrifuged (1,000 rpm, 5 min). The supernatant was removed, PBS was added, and the mixture was centrifuged again (1,000 rpm, 5 min) to remove excess enzyme solution. A pellet was obtained, which was resuspended in a mixed culture medium. Cell counting was performed to obtain the cell concentration of the lung cancer organoid suspension.

Step 3: Human primary lung cancer-associated fibroblasts were taken, digested into single cells, resuspended in the mixed culture medium, and counted.

Step 4: Human umbilical vein endothelial cells HUVEC were taken, digested into single cells, resuspended in the mixed culture medium, and counted.

Step 5: Lung cancer organoid cells, human primary lung cancer-associated fibroblasts, and human umbilical vein endothelial cells were mixed according to a cell number ratio of 1:0.75:0.25 (the total number of cells per well was 5,000, the number of lung cancer organoid cells was 2,500/well, the number of human primary lung cancer-associated fibroblasts was 1,875/well, the number of human umbilical vein endothelial cells was 625/well) to obtain a mixed suspension.

Step 6: The above mixed suspension was added to a low-adhesion U-shaped 96-well plate and centrifuged at 1,000 rpm for 5 min.

Step 7: The plate was placed into an incubator at 37°C with 5% CO₂ for first-stage spheroid culture.

Step 8: After culturing for 2 days, the original culture medium in the well plate was aspirated, 5 µL of matrigel was added. The spheroid was transferred to the middle culture chamber of a drug-sensitive chip. After gelation, 30 µL of mixed culture medium was added to the middle culture chamber of the drug-sensitive chip, and 50 µL of mixed culture medium was added to each of its two side reservoir chambers. The drug-sensitive chip was placed on a rocking perfusion bioreactor, the condition was set to 3°/120 min, and the rocking perfusion bioreactor was placed into an incubator at 37°C with 5% CO₂ for second-stage spheroid culture for 1 day.

The diameter of the vascularized lung cancer organoid spheroid model prepared in Example 2 was 400 µm.

### (II) Drug testing

(1) Drug-containing culture media at appropriate concentrations were prepared: Osimertinib + Bevacizumab (Osimertinib 1 µM, Bevacizumab 1 µg/mL), Docetaxel (3 µM).
(2) The growth state of the vascularized lung cancer organoid spheroid model in the culture chamber of the drug-sensitive chip was observed and recorded. The original culture medium in the culture chamber was aspirated.
(3) 50 µL of drug-containing culture medium was added to each of the two side reservoir chambers of the drug-sensitive chip, and 30 µL of drug-containing culture medium was added to the middle culture chamber.
(4) The drug-loaded drug-sensitive chip was placed on a rocking perfusion bioreactor, the condition was set to 3°/120 min, and the rocking perfusion bioreactor was placed into an incubator at 37°C with 5% CO₂ for dynamic culture.
(5) On Day 3, the growth state of the vascularized lung cancer organoid spheroid model was observed and recorded, and 30 µL of drug-containing culture medium was further added to the middle culture chamber.
(6) On Day 5, the growth state of the vascularized lung cancer organoid spheroid model was observed and recorded, and viability detection was performed using a CTG detection kit.
(7) All drug-containing culture medium was discarded from the two side reservoir chambers, and 20 µL of CTG dilution solution (1:1 dilution of CTG reagent stock solution and mixed culture medium) was added.
(8) The drug-containing culture medium in the middle culture chamber was not discarded; 50 µL of CTG stock solution was directly added and mixed by pipetting.
(9) The chip was shaken on an orbital shaker for 60 minutes to fully lyse the spheroids.
(10) All the lysate was taken out and put into a 96-well plate that was completely opaque on all sides and bottom, and detection was performed using a chemiluminescence microplate reader.

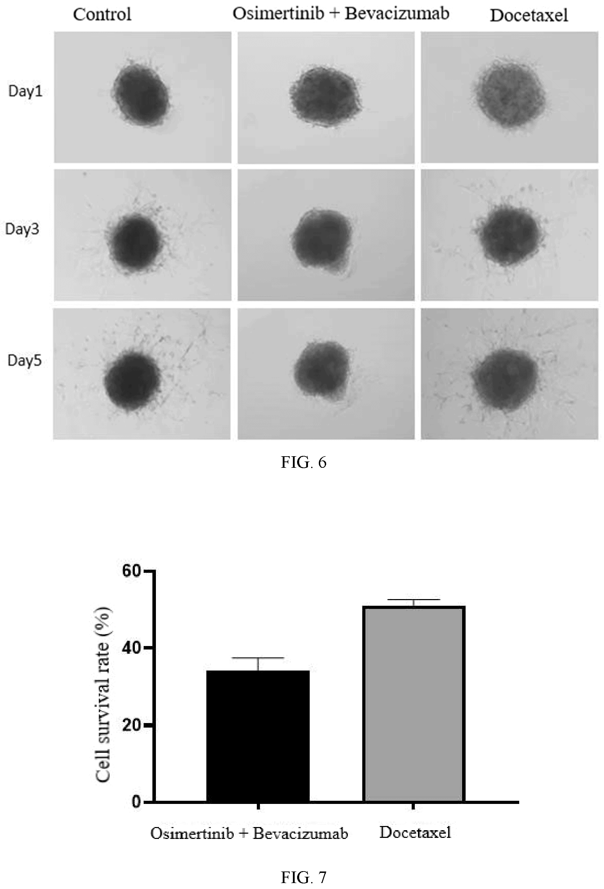

FIG. 6 shows the state of the vascularized lung cancer organoid spheroid model prepared in Example 2 of the present disclosure on Day 3 and Day 5 of culture after adding the drug-containing culture medium during the drug sensitivity test. FIG. 7 is a diagram showing the results of testing the lysates of the three groups of experiments in FIG. 6 using a chemiluminescence microplate reader. Combining FIG. 6 and FIG. 7, it can be seen that using a drug-sensitive chip for dynamic culture, the spheroid model could grow abundant blood vessels. Osimertinib + Bevacizumab (another combination drug) has a significant vascular inhibitory effect on the vascularized lung cancer organoid spheroid model constructed in Example 2 of the present disclosure, while Docetaxel has no obvious vascular inhibitory effect on this sample. At the same time, the combination drug of Osimertinib + Bevacizumab shows stronger sensitivity to this sample.

### Example 3

### (I) Preparation of Vascularized Colorectal Cancer Organoid Spheroid Model

Step 1: A primary colorectal cancer organoid was obtained. Pre-chilled PBS was added to a culture well plate, 1 mL of PBS was added to each well, and the contents were collected into a centrifuge tube. The tube was refrigerated at 4°C for 15 min and then centrifuged (1,000 rpm, 5 min). The supernatant was removed to obtain a colorectal cancer organoid pellet.

Step 2: Tryple E was added for enzymatic digestion for 8 min. After digestion into single cells, the cells were centrifuged (1,000 rpm, 5 min). The supernatant was removed, PBS was added, and the mixture was centrifuged again (1,000 rpm, 5 min) to remove excess enzyme solution. A pellet was obtained, which was resuspended in a mixed culture medium. Cell counting was performed to obtain the cell concentration of the colorectal cancer organoid suspension.

In the mixed culture medium, the mass ratio of the first culture medium to the second culture medium was 1:0.8.

Step 3: Human primary colorectal cancer-associated fibroblasts were taken, digested into single cells, resuspended in the above mixed culture medium, and counted.

Step 4: Human umbilical vein endothelial cells HUVEC were taken, digested into single cells, resuspended in the above mixed culture medium, and counted.

Step 5: Colorectal cancer organoid cells, human primary colorectal cancer-associated fibroblasts, and human umbilical vein endothelial cells were mixed according to a cell number ratio of 34:5:8 (the total number of cells per well was 5,000, the number of colorectal cancer organoid cells was 3,617/well, the number of human primary colorectal cancer-associated fibroblasts was 532/well, the number of human umbilical vein endothelial cells was 851/well) to obtain a colorectal cancer organoid multicellular suspension.

Step 6: The above colorectal cancer organoid multicellular suspension was added to a low-adhesion U-shaped 96-well plate and centrifuged at 1,000 rpm for 5 min.

Step 7: The plate was placed into an incubator at 37°C with 5% CO₂ for first-stage spheroid culture.

Step 8: After culturing for 2 days, the original culture medium in the well plate was aspirated, 5 µL of hydrogel was added. The spheroid was transferred to the middle culture chamber of a drug-sensitive chip. After gelation, 30 µL of mixed culture medium was added to the middle culture chamber of the drug-sensitive chip, and 50 µL of mixed culture medium was added to each of its two side reservoir chambers. The drug-sensitive chip was placed on a rocking perfusion bioreactor, the condition was set to 3°/120 min, and the rocking perfusion bioreactor was placed into an incubator at 37°C with 5% CO₂ for second-stage spheroid culture for 1 day. The diameter of the prepared vascularized colorectal cancer organoid spheroid model was 400 µm.

### (II) Drug sensitivity test

(1) A drug-containing culture medium at an appropriate concentration was prepared: SN-38 (100 nM).
(2) The growth state of the vascularized colorectal cancer organoid spheroid model in the culture chamber of the drug-sensitive chip was observed and recorded. The culture medium in the culture chamber was aspirated.
(3) 50 µL of drug-containing culture medium was added to each of the two side reservoir chambers of the drug-sensitive chip, and 30 µL of drug-containing culture medium was added to the middle culture chamber.
(4) The drug-loaded drug-sensitive chip was placed on a rocking perfusion bioreactor, the condition was set to 3°/120 min, and the rocking perfusion bioreactor was placed into an incubator at 37°C with 5% CO₂ for dynamic culture.
(5) On Day 3, the growth state of the vascularized colorectal cancer organoid spheroid model was observed and recorded, and 30 µL of drug-containing culture medium was further added to the middle culture chamber.
(6) On Day 5, the growth state of the vascularized colorectal cancer organoid spheroid model was observed and recorded, and viability detection was performed using a CTG detection kit.
(7) All drug-containing culture medium was discarded from the two side reservoir chambers, and 20 µL of CTG dilution solution (1:1 dilution of CTG reagent stock solution and mixed culture medium) was added.
(8) The drug-containing culture medium in the middle culture chamber was not discarded; 50 µL of CTG stock solution was directly added and mixed by pipetting.
(9) The chip was shaken on a shaker for 5 min and incubated at room temperature for 25 min.
(10) All the lysate was taken out and put into a 96-well plate that was completely opaque on all sides and bottom, and detection was performed using a chemiluminescence microplate reader.

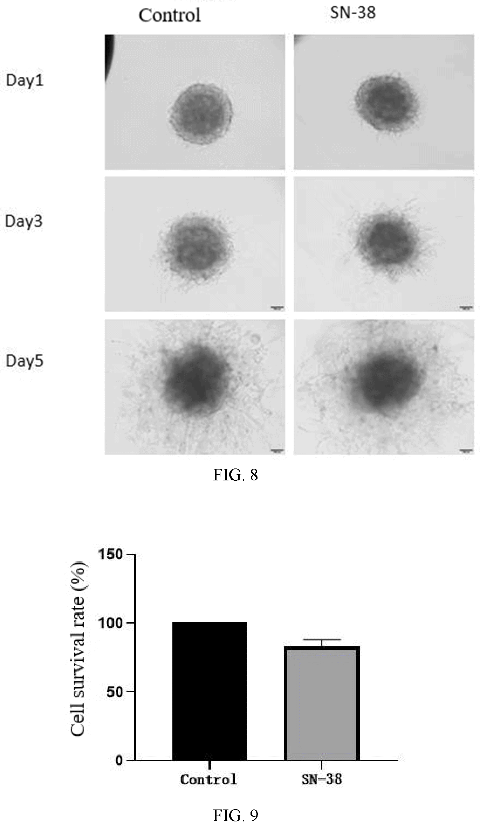

FIG. 8 shows the state of the vascularized colorectal cancer organoid spheroid model prepared in Example 3 of the present disclosure on Day 3 and Day 5 of culture after adding the drug-containing culture medium during the drug sensitivity test. FIG. 9 is a diagram showing the results of testing the lysates in FIG. 8 using a chemiluminescence microplate reader. Combining FIG. 8 and FIG. 9, it can be seen that the inhibition rate of SN-38 on the cell viability of this colorectal cancer organoid spheroid is 17%, and the inhibitory effect is not significant. Here, "control" refers to the cell viability of the control group (the same applies below).

### Example 4

### (I) Preparation of Vascularized Pancreatic Cancer Organoid Spheroid Model

Step 1: A primary pancreatic cancer organoid was obtained. Pre-chilled PBS was added to a culture well plate, 1 mL of PBS was added to each well, and the contents were collected into a centrifuge tube. The tube was refrigerated at 4°C for 15 min and then centrifuged (1,000 rpm, 5 min). The supernatant was removed to obtain a pancreatic cancer organoid pellet.

Step 2: Tryple E was added for enzymatic digestion for 8 min. After digestion into single cells, the cells were centrifuged (1,000 rpm, 5 min). The supernatant was removed, PBS was added, and the mixture was centrifuged again (1,000 rpm, 5 min) to remove excess enzyme solution. A pellet was obtained, which was resuspended in a mixed culture medium. Cell counting was performed to obtain the cell concentration of the pancreatic cancer organoid suspension.

In the mixed culture medium, the mass ratio of the first culture medium to the second culture medium was 1:1.2.

Step 3: Human pancreatic cancer-associated immortalized fibroblasts were taken, digested into single cells, resuspended in the above mixed culture medium, and counted.

Step 4: Human umbilical vein endothelial cells HUVEC were taken, digested into single cells, resuspended in the above mixed culture medium, and counted.

Step 5: Pancreatic cancer organoid cells, human pancreatic cancer-associated immortalized fibroblasts, and human umbilical vein endothelial cells were mixed according to a cell number ratio of 2:1:1 (the total number of cells per well was 5,000, the number of pancreatic cancer organoid cells was 2,500/well, the number of human pancreatic cancer-associated immortalized fibroblasts was 1,250/well, the number of human umbilical vein endothelial cells was 1,250/well) to obtain a pancreatic cancer organoid multicellular suspension.

Step 6: The above pancreatic cancer organoid multicellular suspension was added to a low-adhesion U-shaped 96-well plate and centrifuged at 1,000 rpm for 5 min.

Step 7: The plate was placed into an incubator at 37°C with 5% CO₂ for first-stage spheroid culture.

Step 8: After culturing for 2 days, the original culture medium in the well plate was aspirated, 5µL of hydrogel was added. The spheroid was transferred to the middle culture chamber of a drug-sensitive chip. After gelation, 30 µL of mixed culture medium was added to the middle culture chamber of the drug-sensitive chip, and 50 µL of mixed culture medium was added to each of its two side reservoir chambers. The drug-sensitive chip was placed on a rocking perfusion bioreactor, the condition was set to 3°/120 min, and the rocking perfusion bioreactor was placed into an incubator at 37°C with 5% CO₂ for second-stage spheroid culture for 1 day. The diameter of the prepared vascularized pancreatic cancer organoid spheroid model was 400 µm.

### (II) Drug sensitivity test:

(1) A drug-containing culture medium at an appropriate concentration was prepared: Gemcitabine (10 µM).
(2) The growth state of the vascularized pancreatic cancer organoid spheroid model in the culture chamber of the drug-sensitive chip was observed and recorded. The culture medium in the culture chamber was aspirated.
(3) 50 µL of drug-containing culture medium was added to each of the two side reservoir chambers of the drug-sensitive chip, and 30 µL of drug-containing culture medium was added to the middle culture chamber.
(4) The drug-loaded drug-sensitive chip was placed on a rocking perfusion bioreactor, the condition was set to 3°/120 min, and the rocking perfusion bioreactor was placed into an incubator at 37°C with 5% CO₂ for dynamic culture.
(5) On Day 3, the growth state of the vascularized pancreatic cancer organoid spheroid model was observed and recorded, and 30 µL of drug-containing culture medium was further added to the middle culture chamber.
(6) On Day 5, the growth state of the vascularized pancreatic cancer organoid spheroid model was observed and recorded, and viability detection was performed using a CTG detection kit.
(7) All drug-containing culture medium was discarded from the two side reservoir chambers, and 20 µL of CTG dilution solution (1:1 dilution of CTG reagent stock solution and mixed culture medium) was added.
(8) The drug-containing culture medium in the middle culture chamber was not discarded; 50 µL of CTG stock solution was directly added and mixed by pipetting.
(9) The chip was shaken on a shaker for 5 min and incubated at room temperature for 25 min.
(10) All the lysate was taken out and put into a 96-well plate that was completely opaque on all sides and bottom, and detection was performed using a chemiluminescence microplate reader.

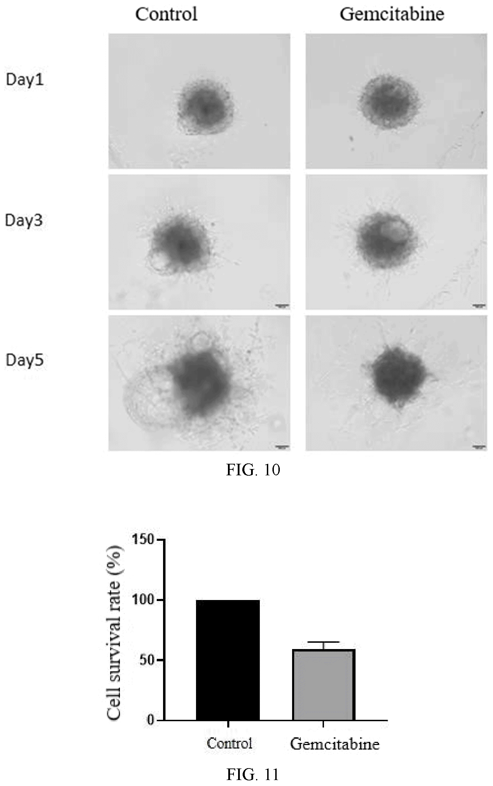

FIG. 10 shows the state of the vascularized pancreatic cancer organoid spheroid model prepared in Example 4 of the present disclosure on Day 3 and Day 5 of culture after adding the drug-containing culture medium during the drug sensitivity test. FIG. 11 is a diagram showing the results of testing the lysates in FIG. 10 using a chemiluminescence microplate reader. Combining FIG. 10 and FIG. 11, it can be seen that the inhibition rate of Gemcitabine on the cell viability of this pancreatic cancer organoid spheroid is 40%, but Gemcitabine has no obvious inhibitory effect on vascular growth.

### Example 5

### Preparation of Vascularized Liver Organoid Spheroid Model

Step 1: A primary liver organoid was obtained. Pre-chilled PBS was added to a culture well plate, 1 mL of PBS was added to each well, and the contents were collected into a centrifuge tube. The tube was refrigerated at 4°C for 15 min and then centrifuged (1,000 rpm, 5 min). The supernatant was removed to obtain a liver organoid pellet.

Step 2: Tryple E was added for enzymatic digestion for 8 min. After digestion into single cells, the cells were centrifuged (1,000 rpm, 5 min). The supernatant was removed, PBS was added, and the mixture was centrifuged again (1,000 rpm, 5 min) to remove excess enzyme solution. A pellet was obtained, which was resuspended in a mixed culture medium. Cell counting was performed to obtain the cell concentration of the liver organoid suspension.

In the mixed culture medium, the mass ratio of the first culture medium to the second culture medium was 1:0.8.

Step 3: Human primary liver-associated fibroblasts were taken, digested into single cells, resuspended in the above mixed culture medium, and counted.

Step 4: Human umbilical vein endothelial cells HUVEC were taken, digested into single cells, resuspended in the above mixed culture medium, and counted.

Step 5: Liver organoid cells, human primary liver-associated fibroblasts, and human umbilical vein endothelial cells were mixed according to a cell number ratio of 2:3:3 (the total number of cells per well was 5,000, the number of liver organoid cells was 1250/well, the number of human primary liver fibroblasts was 1,875/well, the number of human umbilical vein endothelial cells was 1,875/well) to obtain a mixed suspension.

Step 6: The above mixed suspension was added to a low-adhesion U-shaped 96-well plate and centrifuged at 1,000 rpm for 5 min.

Step 7: The plate was placed into an incubator at 37°C with 5% CO₂ for first-stage spheroid culture.

Step 8: After culturing for 2 days, the original culture medium in the well plate was aspirated, 5 µL of hydrogel was added. The spheroid was transferred to the middle well of a drug-sensitive chip. After gelation, 30 µL of mixed culture medium was added. 50 µL of mixed culture medium was added to each of the two side wells of the drug-sensitive chip. The drug-sensitive chip was placed on a rocking perfusion bioreactor. The condition was set to 3°/120 min. The rocking perfusion bioreactor was placed into an incubator at 37°C with 5% CO₂ for second-stage spheroid culture for 1 day. The diameter of the prepared vascularized liver organoid spheroid model was 400 µm.

### Hepatotoxicity test:

(1) Drug-containing culture media at appropriate concentrations were prepared: Clozapine (2.4 µM), Troglitazone (6.1 µM).
(2) The growth state of the vascularized liver organoid spheroid model in the culture chamber of the drug-sensitive chip was observed and recorded. The culture medium in the culture chamber was aspirated.
(3) 50 µL of drug-containing culture medium was added to each of the two side reservoir chambers of the drug-sensitive chip, and 30 µL of drug-containing culture medium was added to the middle culture chamber.
(4) The drug-loaded drug-sensitive chip was placed on a rocking perfusion bioreactor, the condition was set to 3°/120 min, and the rocking perfusion bioreactor was placed into an incubator at 37°C with 5% CO₂ for dynamic culture.
(5) On Day 3, the growth state of the vascularized liver organoid spheroid model was observed and recorded, and 30 µL of drug-containing culture medium was further added to the middle culture chamber.
(6) On Day 5, the growth state of the vascularized liver organoid spheroid model was observed and recorded, and viability detection was performed using a CTG detection kit.
(7) All drug-containing culture medium was discarded from the two side reservoir chambers, and 20 µL of CTG dilution solution (1:1 dilution of CTG reagent stock solution and culture medium) was added.
(8) The drug-containing culture medium in the middle culture chamber was not discarded; 50 µL of CTG stock solution was directly added and mixed by pipetting.
(9) The chip was shaken on a shaker for 5 min and incubated at room temperature for 25 min.
(10) All the lysate was taken out and put into a 96-well plate that was completely opaque on all sides and bottom, and detection was performed using a chemiluminescence microplate reader.

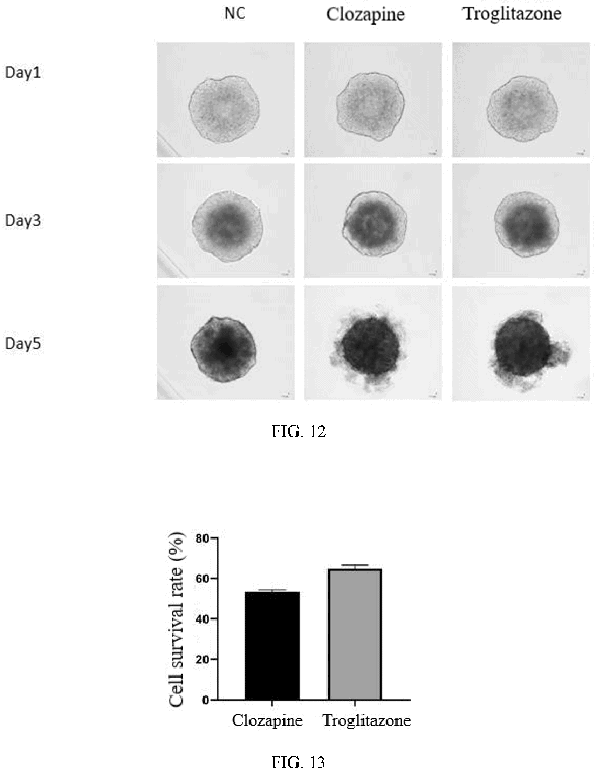

FIG. 12 shows the state of the vascularized liver organoid spheroid model prepared in Example 5 of the present disclosure on Day 3 and Day 5 of culture after adding the drug-containing culture medium during the hepatotoxicity test, wherein NC is the blank control group. FIG. 13 is a diagram showing the results of testing the lysates of the three groups of experiments in FIG. 12 using a chemiluminescence microplate reader. Combining FIG. 12 and FIG. 13, it can be seen that both Clozapine and Troglitazone have a certain degree of damage to the liver organoid, especially Clozapine has a stronger toxic effect on the liver organoid.

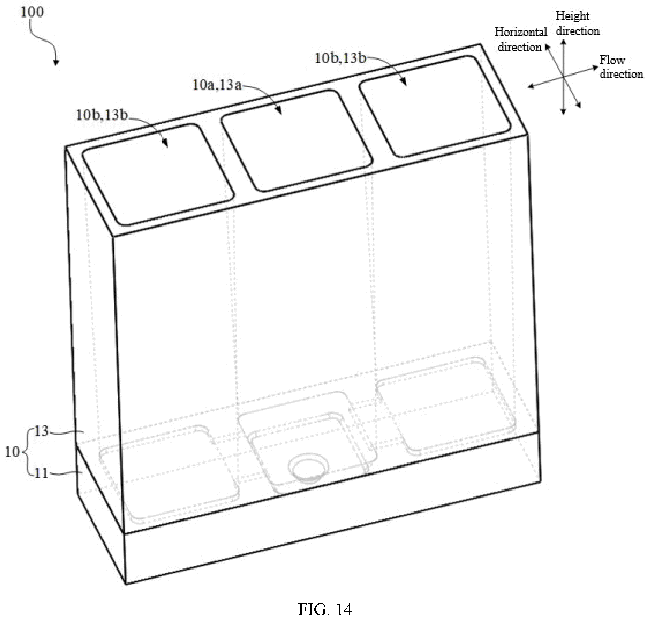

The drug-sensitive chip used in the present disclosure employs, for example, the cell culture device shown in patent applications CN2023105307566, CN2023211335836, CN2023105302350, and CN202310530706.8. Exemplarily, taking the cell culture device shown in patent application CN202310530706.8 as an example and referring to FIG. 14, the middle culture chamber of the drug-sensitive chip of the present disclosure referred to the culture chamber 10a in the cell culture device, and the two side reservoir chambers of the drug-sensitive chip of the present disclosure referred to the reservoir chambers 10b in the cell culture device. The number of reservoir chambers 10b is two, and in the flow direction from the reservoir chambers 10b to the culture chamber 10a, the two reservoir chambers 10b are arranged symmetrically, and the culture chamber 10a is located between the two reservoir chambers 10b. With this arrangement, the cell culture device can be rocked. At this time, under rocking, the culture medium can move back and forth between the two reservoir chambers 10b, generating physicochemical stimuli such as fluid shear stress, mechanical stress, and biochemical concentration gradients to achieve dynamic culture. While exhibiting more realistic physiological functions, it can better achieve material exchange between the culture medium and the biological sample, and can also take away the waste products of the biological sample, thus better simulating the real cellular environment.

The above are only preferred examples of the present disclosure and are not intended to limit the present disclosure. Any modifications, equivalent substitutions and improvements made within the spirit and principles of the present disclosure should be included within the protection scope of the present disclosure.

## Claims

1. A method for constructing a vascularized organoid spheroid model, comprising:
S100: preparing or obtaining an organoid, wherein the organoid is a primary organoid or a passaged organoid, digesting the primary organoid or the passaged organoid to obtain organoid cells; and
obtaining fibroblasts and vascular endothelial cells;
S200: mixing the organoid cells, the fibroblasts and the vascular endothelial cells, and resuspending them with a culture medium to obtain an organoid multicellular suspension; and
S300: inoculating the organoid multicellular suspension into a culture chamber or a culture well, and performing spheroid culture, allowing self-assembly to form the vascularized organoid spheroid model.

2. The method according to claim 1, wherein the organoid is a primary tumor organoid or a passaged tumor organoid;
the primary tumor organoid or the passaged tumor organoid is digested into single cells to obtain tumor organoid cells;
the fibroblasts comprise tumor-associated fibroblasts; and
a cell number ratio of the tumor organoid cells, the tumor-associated fibroblasts and the vascular endothelial cells is 1: (0.1-1): (0.05-1).

3. The method according to claim 1, wherein the culture medium is a mixed culture medium;
the mixed culture medium comprises at least a first culture medium for culturing the organoid cells and a second culture medium for culturing the vascular endothelial cells; and
in the mixed culture medium, a mass ratio of the first culture medium to the second culture medium is 1: (0.5-2).

4. The method according to claim 3, wherein in step S300, when the organoid multicellular suspension is inoculated into a culture chamber, the culture chamber has a U-shaped bottom; or
when the organoid multicellular suspension is inoculated into a culture well, the culture well has a U-shaped bottom.

5. The method according to claim 4, wherein the spheroid culture comprises a first-stage spheroid culture and a second-stage spheroid culture; and
the first-stage spheroid culture comprises: inoculating the organoid multicellular suspension into the culture chamber or the culture well for aggregation treatment to aggregate the cells in the organoid multicellular suspension, and then performing culture for 1 to 5 days after placement in a culture environment, to obtain an organoid spheroid model intermediate with clear boundaries and microvessels, enabling the second-stage spheroid culture to be performed subsequently.

6. The method according to claim 5, wherein the second-stage spheroid culture comprises: discarding an original culture medium in the culture chamber or the culture well containing the organoid spheroid model intermediate, adding a cell culture scaffold material, adding the mixed culture medium after gelation, and performing culture for 1 to 2 days after placement in a culture environment.

7. The method according to claim 5, wherein the second-stage spheroid culture comprises: discarding an original culture medium in the culture chamber or the culture well containing the organoid spheroid model intermediate, adding a cell culture scaffold material, transferring a resulting mixture to a culture chamber of a drug-sensitive chip or a culture well of a drug-sensitive chip, adding the mixed culture medium after gelation, and performing dynamic culture for 1 to 2 days after placement in a culture environment; and
the culture chambers of the drug-sensitive chip and the culture wells of the drug-sensitive chip have U-shaped bottoms and are interconnected.

8. The method according to claim 7, wherein the dynamic culture comprises:
performing flow perfusion culture at a speed of rocking 3° to 10° every 15 min to 120 min in the culture environment.

9. The method according to claim 2, wherein the organoid is a primary lung cancer organoid, a lung cancer organoid differentiated from iPS stem cells or a passaged lung cancer organoid;
the primary lung cancer organoid, the lung cancer organoid differentiated from iPS stem cells or the passaged lung cancer organoid is digested into single cells to obtain lung cancer organoid cells;
the fibroblasts comprise lung cancer-associated fibroblasts;
a cell number ratio of the lung cancer organoid cells, the lung cancer-associated fibroblasts and the vascular endothelial cells is 1: (0.4-0.8): (0.1-0.5); and
preferably, a total cell count of the lung cancer organoid cells, the fibroblasts and the vascular endothelial cells ranges from 5,000 to 2×10⁴.

10. The method according to claim 2, wherein the organoid is a primary colorectal cancer organoid or a passaged colorectal cancer organoid;
the primary colorectal cancer organoid or the passaged colorectal cancer organoid is digested into single cells to obtain colorectal cancer organoid cells;
the fibroblasts comprise colorectal cancer-associated fibroblasts; and
a cell number ratio of the colorectal cancer organoid cells, the colorectal cancer-associated fibroblasts and the vascular endothelial cells is 1: (0.1-0.8) : (0.2-0.6).

11. The method according to claim 2, wherein the organoid is a primary pancreatic cancer organoid or a passaged pancreatic cancer organoid;
the primary pancreatic cancer organoid or the passaged pancreatic cancer organoid is digested into single cells to obtain pancreatic cancer organoid cells;
the fibroblasts comprise pancreatic cancer-associated fibroblasts; and
a cell number ratio of the pancreatic cancer organoid cells, the pancreatic cancer-associated fibroblasts and the vascular endothelial cells is 1: (0.2-0.7) : (0.2-0.7).

12. The method according to claim 1, wherein the organoid is a primary non-tumor organoid or a passaged non-tumor organoid;
the primary non-tumor organoid or the passaged non-tumor organoid is digested into single cells to obtain non-tumor organoid cells;
the non-tumor organoid is selected from any one of a liver organoid, a lung organoid, a colorectal organoid or a pancreatic organoid; and
the fibroblasts comprise relevant fibroblasts.

13. A vascularized organoid spheroid model, wherein the vascularized organoid spheroid model has a spheroid structure, with a plurality of organoids aggregated and growing inside;
the vascularized organoid spheroid model has microvessels growing divergently from inside to outside;
the vascularized organoid spheroid model further comprises an organoid microenvironment composed of fibroblast structures and microvessels; and
the vascularized organoid spheroid model has a diameter ranging from 250 µm to 800 µm.

14. The vascularized organoid spheroid model according to claim 13, wherein the vascularized organoid spheroid model has cell proliferation and an extent of cell proliferation increases in a gradient from inside to outside; and
an oxygen content of the vascularized organoid spheroid model gradually increases from inside to outside.

15. The vascularized organoid spheroid model according to claim 13, wherein the vascularized organoid spheroid model is constructed according to the method of any one of claims 1 to 12.

16. Use of the vascularized organoid spheroid model according to any one of claims 13 to 15 in testing; and
the testing comprises any one of drug screening, drug sensitivity testing, drug toxicity testing or non-drug substance toxicity testing.

17. The use according to claim 16, comprising at least:
adding a drug to be tested to a culture chamber or a culture well containing the vascularized organoid spheroid model, performing culture for 3 to 5 days after placement in a culture environment, and detecting a change in an indicator of the vascularized organoid spheroid model; or
adding a drug to be tested to a culture chamber of a drug-sensitive chip or a culture well of a drug-sensitive chip containing the vascularized organoid spheroid model, performing dynamic culture for 3 to 5 days after placement in a culture environment, and detecting a change in an indicator of the vascularized organoid spheroid model; and
wherein the change in the indicator comprises at least one of a change in shape, a change in size, a change in viability, a change in vascular morphology, a change in protein molecular expression or a change in distribution of the vascularized organoid spheroid model.

18. A drug testing method, comprising:
S100: preparing or obtaining an organoid, digesting the organoid to obtain organoid cells, and obtaining fibroblasts and vascular endothelial cells;
S200: mixing the organoid cells, the fibroblasts and the vascular endothelial cells, and resuspending them with a culture medium to obtain an organoid multicellular suspension;
S300: inoculating the organoid multicellular suspension into a culture chamber or a culture well, and performing spheroid culture, allowing self-assembly to form a vascularized organoid spheroid model; and
S400: adding a drug to be tested to the culture chamber or the culture well containing the vascularized organoid spheroid model, performing culture after placement in a culture environment, and then detecting a change in an indicator of the vascularized organoid spheroid model;
preferably, the organoid is as defined in any one of claims 1 to 2 and 9 to 12; the fibroblasts comprise tumor-associated fibroblasts; a cell number ratio of the tumor organoid cells, the tumor-associated fibroblasts and the vascular endothelial cells is 1: (0.1-1): (0.05-1); and
preferably, the spheroid culture is as defined in any one of claims 5 to 8.

19. A culture medium composition, wherein the culture medium composition comprises a first culture medium for culturing organoid cells and a second culture medium for culturing vascular endothelial cells, and a mass ratio of the first culture medium to the second culture medium is 1: (0.5-2);
preferably, the culture medium composition is used in the method of any one of claims 1 to 12, or used in the construction of the vascularized organoid spheroid model of any one of claims 13 to 15;
preferably, the mass ratio of the first culture medium to the second culture medium is 1: (0.8-1.2); and
preferably, the organoid cells are as defined in any one of claims 1 to 2 and 9 to 12.
